# EUROPEAN PATENT SPECIFICATION

(11) **EP 2 313 356 B1**
(45) Date of publication and mention of the grant of the patent: **26.03.2014**
(21) Application number: 08783225.9
(22) Date of filing: 16.07.2008
(51) Int. Cl.: C07C 41/09, C07C 41/01, C07C 43/10, C07C 43/13, C07C 43/15, C10L 1/185

(54) **CONVERSION OF GLYCEROL TO NAPHTHA-RANGE OXYGENATES**
UMWANDLUNG VON GLYCERIN IN OXYGENATE DES NAPHTHABEREICHS
CONVERSION DU GLYCEROL EN COMPOSES OXYGENES DE TYPE NAPHTA

(43) Date of publication of application: 27.04.2011
(73) Proprietor: Her Majesty The Queen In Right Of Canada As Represented By The Minister Of Natural Resources Canada, Ottawa, Ontario K1A 0E4 (CA)
(72) Inventor: IKURA, Michio, Kanata Ontario K2K 1P4 (CA)
(74) Representative: Potter Clarkson LLP
(86) International application number: PCT/CA2008/001309
(87) International publication number: WO 2010/006402

(56) References cited:
- WO-A1-2006/084048
- WO-A1-2007/027669
- WO-A1-2007/043063
- WO-A2-2007/113776
- WO-A2-2008/052993
- GB-A- 1 047 214
- US-A- 6 113 661
- MAYS M.A.: 'The use of oxygenated hydrocarbons in gasoline and their contribution to reducing urban air pollution' PURE & APPL. CHEM. vol. 61, no. 8, 1989, pages 1373 - 1378, XP008142393

## Description

### TECHNICAL FIELD

This invention relates to the conversion of glycerol to products of commercial utility. More particularly, the invention relates to methods of conversion that produce fuels and, especially, fuel additives.

### BACKGROUND ART

Bio-diesel production by the trans-esterification of triglycerides with alcohol produces glycerol (1,2,3-propane triol) as a by-product. Triglycerides commonly used for the process include animal fats and vegetable oils, such as canola oil and soybean oil. Bio-diesel production has been increasing worldwide but glycerol utilization and processing is lagging. Glycerol can be processed to high value products such as medicinal grade glycerine and animal feeds. However, such uses of and markets for glycerol are well established and well supplied, so the continued production of glycerol as a by-product of bio-diesel production would have a major effect on the price stability of glycerol markets. Moreover, the glycerol produced as a by-product of bio-diesel production is generally impure, which further reduces its commercial value. It is therefore desirable to develop new products from glycerol that have value in other markets.

Fuels of all kinds, and particularly those for internal combustion engines, are in increasingly high demand nowadays, so it would be desirable to utilize glycerol or its products as a fuel or fuel additive. While glycerol may be co-combusted with biomass as a fuel extender for boilers and heaters, it is not itself suitable for use with internal combustion engines. For instance, unlike gasoline, glycerol does not evaporate into an engine head under slight suction, making it unusable as a gasoline additive. Further, glycerol cannot be mixed with diesel fuel due to its high polarity; when the mixing of the two is attempted, glycerol separates from the diesel and settles as a lower layer. Glycerol may act as a surfactant when mixed with bio-diesel and water to produce relatively stable water-in-biodiesel emulsions. However, when such emulsions are combusted in a diesel engine, acrolein levels in the exhaust become unacceptably high when the amount of glycerol in the fuel is higher than 0.5 vol.% (see Hamasaki, K., Kinoshita, E., Tajima, H., Takasaki, K., Morita, D., "Combustion Characteristics of Diesel Engines with Waste Vegetable Oil Methyl Esters", The Fifth International Symposium on Diagnostics of Combustion in Internal Combustion Engines (COMODIA 2001), July 1-4, 2001, Nagoya, Japan).

WO2007/113776 discloses the conversion of glycerol to oxygenates using lower alcohols in the presence of a solid acid catalyst and high pressure.

There is therefore a need for new products from glycerol that have commercial value, especially as fuels or fuel additives.

### DISCLOSURE OF THE INVENTION

According to one exemplary embodiment of the invention, there is provided a method of converting glycerol to one or more oxygenates of lower boiling point than glycerol itself. The method comprises reacting glycerol with a lower alcohol in the vapor phase under reduced pressure at a reaction temperature in a range of 150 to 300°C in the presence of a solid acidic catalyst.

The term "lower alcohol" means an alcohol selected from the group consisting of methanol, ethanol. straight chained or branched propanol, straight chained or branched butanol and mixtures of any two or more thereof.

Also disclosed herein are oxygenates produced by the above method, and fuel additives for gasoline containing the oxygenates.

The term "oxygenates" is generally understood to mean oxygenated chemical compounds that contain oxygen as a part of their chemical structure, and, most preferably in the context of this invention, oxygenated hydrocarbons that contain only carbon, hydrogen and oxygen). The term may be referred to oxygenated fuels and more especially oxygenated fuel additives. Oxygenates are often employed as gasoline additives to reduce levels of carbon monoxide that is created during the burning of the gasoline. The use of oxygenates is explained more fully, for example, in an article by M.A. Mays, "The use of Oxygenated Hydrocarbons in Gasoline and Their Contribution to Reducing Urban Air Pollution", Pure & Appl.Chem. Vol 61, No. 8, pp. 1373-1378, 1989, and also in U.S. Patent 6,623,535 issued on September 23, 2003 to Horst Kief.

### BRIEF DESCRIPTION OF THE DRAWINGS

The present invention will now be described in further detail with reference to the accompanying drawing, in which:

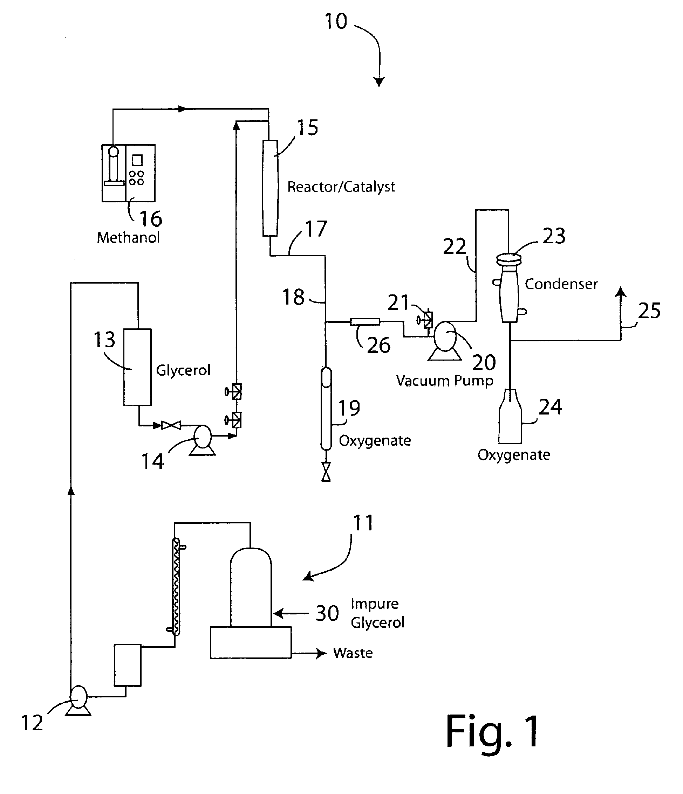

Fig. 1 is a diagram illustrating apparatus suitable for carrying out an exemplary embodiment of the method of the invention.

### DETAILED DESCRIPTION OF THE EXEMPLARY EMBODIMENTS

Exemplary embodiments of the invention provide methods of converting glycerol to products that have value as fuels and, especially, fuel additives. A preferred exemplary embodiment involves reacting glycerol with one or more lower alcohols under conditions that produce volatile oxygenates, particularly those having boiling points lower than glycerol itself. i.e. lower than 290°C at atmospheric pressure. Ideally, the products have boiling points within or near the naphtha range, e.g. from about 38°C to about 250°C, more preferably within the range of about 149°C to 221°C. and ideally below 204°C. Initial boiling points (IBPs) of about 165°C and below are preferably targeted.

The conversion reaction is carried out entirely in the gaseous phase, and reduced pressure is generally required to vaporize the reactants (both glycerol and the alcohol) at a suitable reaction temperature. The actual reduced pressure that is required depends on the desired reaction temperature and, to some extent on the reactants (e.g. the alcohol selected for reaction). In general, a reaction temperature in the range of 150 to 300°C is employed, and more preferably 200 to 250°C. The pressure is normally kept below about 2.2 psia (115 mm Hg). For example, the pressure may be below about 0.2 psia (11mm Hg) when the reaction temperature is about 200°C, and below about 2.2 psia (115 mm Hg) when the reaction temperature is about 250°C. As an example, when glycerol is reacted with methanol at a temperature in the range of 200 to 250°C, a pressure of 0.1 psia (5mm Hg) or less is generally required to ensure that both reactants are in the vapor phase.

The reaction is carried out over a solid acidic catalyst, preferably a solid superacid catalyst, such as zirconium sulphate on titanium oxide (TiO₂/Zr(SO₄)₂), sulphated tin oxide (SnO₂/SO₄²⁻), or sulphated zirconium oxide (ZrO₂/ SO₄²⁻). Such catalysts may be produced, for example, by calcining sulphated zirconium hydroxide to form a ZrO₂/SO₄²⁻ superacid catalyst.

It is preferable to use the catalyst as solid extrudates rather than fine particles in order to reduce the pressure drop within the reactor. When a fine power is used, it may sometimes not be possible to create a sufficiently high vacuum in the reactor, somewhat depending on the dimensions of the reactor. An extrudate sized to produce a predetermined pressure drop for a given reactor (e.g. an elongated tubular reactor) is preferably employed.

The alcohol used as a feedstock is most preferably methanol, but may be ethanol, propanol or butanol. The propanol and butanol may be either straight chained or branched. If alcohols other than methanol are employed, the majority of the resulting oxygenates will be slightly heavier than those obtained with methanol due to the increased carbon number of these reactants, but products useful as fuels and fuel additives are still produced. A mixture of two or more alcohols may be used as a feedstock, if desired, but no significant advantage is thereby obtained. The alcohol is normally present in weight excess, e.g. preferably a ratio of at least 2:1 by weight, and more preferably up to about 4:1 by weight. The alcohols may be obtained from common commercial sources and may be of plant origin.

Glycerol from any source may be employed as a feedstock, but it is preferred to use glycerol produced as a by-product of the trans-esterification of triglycerides due to the economic incentive mentioned above. The glycerol must be free or substantially free of inorganics (e.g. metal atoms or ions) as they tend to foul the catalyst surface and may result in catalyst deactivation. Glycerol obtained as byproducts of commercial trans-esterification are normally contaminated with a catalyst (KOH. NaOH, CH₃ONa - sodium methoxide, etc.) and methanol. Such feedstocks should first be purified, e.g. by vacuum distillation.

Reaction times are decided with a view to converting as much of the glycerol to oxygenates as possible. In practice, reaction times (reaction residence times) are preferably in the range of 5 - 50 minutes, and are often 10 minutes or less.

Under vapor phase conditions, glycerol conversion proceeds very rapidly and fairly selectively to produce mostly light oxygenates (normally mixtures of ethers, esters, aldehydes and ketones). Glycerol is a triol and can react with alcohols under the reaction conditions in various ways, e.g. by esterification, etherification (alkylation), etc. The reaction products may include, for example, water and light oxygenates such as, for example:
**3-methoxy 1-propene** [CH₃CH=CHOCH₃, b.p. 35°C],
**acrolein dimethyl acetal** [H₂C=CHCH(OCH₃)₂, b.p. 90°C], **1,3-trimethoxypropane** [(CH₃O)₂CHCH₂CH₂OCH₃, b.p. 145°C (estimate),
or 45-46°C at 17mm Hg], and/or
**1,3-dimethoxy 2-propanol** [H₃COCH₂CHOHCH₂OCH₃, b.p. 169°C].

The reaction products are generally further processed before use. For example, water may be stripped from the product, as well as unreacted alcohol for reuse in the vapour phase reaction, by means of rectification (precise distillation). Any unpleasant or noxious odors in the product stream are generally removed during the rectification process. Alternatively, water may be removed by passing the product stream through water absorption media (e.g. Molecular Sieve 3). In general, the individual oxygenates do not have to be separated from each other prior to use, although they may be, if desired. The reaction products may be used as high octane gasoline additives in the same manner as methanol and ethanol themselves, but with higher fuel values. The reaction products may be mixed with other compatible fuel additives, if desired.

The oxygenates produced by the method of the exemplary embodiments are suitable for use as fuel additives because they mix readily with gasoline and vaporize readily during the combustion process. They contain both oxygen, that may convert carbon monoxide to carbon dioxide during the combustion of the fuel, and carbon, that is combusted and adds to the heat and gases generated by the fuel. The amounts in which the oxygenates should be used with any particular fuel will be well known to persons skilled in the art.

A representation of a preferred embodiment of the method and corresponding apparatus is provided in Fig. 1 of the accompanying drawings. In the figure, the apparatus is shown generally at 10. Impure glycerol 30 (e.g. a by-product from the trans-esterification of triglycerides) is purified by vacuum distillation in a distillation apparatus 11 and is then supplied by pump 12 to a heated and insulated tank 13. From the tank 13, the heated glycerol is pumped via a gear pump 14 to a tubular reactor 15 filled with solid extrudates of an acidic esterification catalyst. Methanol is supplied from a syringe pump 16 in a suitable ratio relative to the amount of glycerol. The glycerol and methanol are mixed together immediately ahead of the reactor due to the joining of the supply pipes, as shown. It would also be possible to premix the glycerol and methanol in an additional vessel in a predetermined ratio and then feed the mixture to the reactor 15. The reactor 15 is heated (e.g. via external heating tape) and insulated in order to ensure that the target reaction temperature is attained within the reactor. The reactor 15 is also maintained under sufficiently low pressure via a vacuum pump 20 to vaporize the glycerol and methanol in contact with the catalyst. The vacuum pump is preferably one designed to operate at high temperatues, e.g. more than 100°C. An in-line heater 26 may be provided immediately upstream of the vacuum pump 20 to prevent condensation of vapor within the pump itself.

The reaction products leave the reactor 15 via delivery pipe 17 and condensation of high boiling liquids may take place in a vertical stretch 18 of this pipe. The liquid condensed in this way is collected in metal container 19. Air may be introduced into the system in measured amounts via an air bleed valve 21 and air and remaining vapor is expelled from the vacuum pump 20 via delivery pipe 22. Remaining vapor may be condensed in a condenser 23 and collected in a container 24. Residual gas is expelled via pipe 25. If the expelled gas contains further product vapor, still further condensers may be provided downstream, including liquid nitrogen traps, if needed.

The exemplary embodiments of the present invention arc illustrated further by way of the following Examples, which should not be considered as limiting the scope of the present invention. The Examples employ methanol as the alcohol used for the conversion reaction, but this is merely representative of the lower alcohols that may be employed and that all work in essentially the same way as they are all fairly volatile short chain reactants containing the -OH group.

### EXAMPLES

### Synthesis Of Catalysts

### TiO₂/Zr(SO₄)₂ Catalyst

The procedure followed was that of Sohn, J.R., Lee, D.G. (2003) "Characterization of Zirconium Sulfate Supported on TiO2 and Activity for Acid Catalysis", Korean Journal of Chemical Engineering, 20 (6) September 2003, pp. 1030-1036.

Zr(SO₄)₂ (6.72g) was dissolved in a suitable amount of water (51.42 g) and this solution was added to TriO₂ powder (18.52 g), which was then stirred in a glass bottle for 8 hours.

The solution was dried in an oven at 120°C for an additional 4 hours before being calcined at 400°C for 2 hours.

The catalyst was broken into pieces and screened to ensure particle sizes were greater than 18 mesh.

### SnO₂/SO₄²⁻ Catalyst

The procedure followed was that of Furuta, S.. Matsuhashi, H., Arata, K. (2004) "Catalytic Action Of Sulphated Tin Oxide For Etherification And Esterification In Comparison With Sulfated Zirconia." Applied Catalysis A: General, 269 April, pp. 187-191.

SnCl₄ was sampled by opening the seal of a bottle containing the substance and purging the top of the bottle with nitrogen while extracting the substance with a syringe equipped with Tygon^{®} tubing rather than a syringe tip. The extracted SnCl₄ was diluted in water (94.9g SnCl₄ in 3L of water).

30% ammonium hydroxide solution was added dropwise with stirring to the SnCl₄ solution until the pH reached 8. which was approximately 150 mL. whereby a white precipitate was observed.

The precipitate was filtered with # 42 ashless filter paper, using water aspiration for the suction of the filtration.

The resulting filtered 'gel like' product was removed whenever the filter became clogged and the filtration slowed down. The product was placed in 4 L of 4% ammonium acetate solution (133.2 g of glacial acetic acid and 260.9 g of 30% ammonium hydroxide solution diluted to 4L with water) until all filtering was complete (left overnight in solution).

After letting the gel sit in ammonium acetate solution, the solution was suction filtered again with # 42 ashless filter paper and dried at 110°C for 24 hours.

The dried wax-like product was then stirred for 1 hour in a glass flask with 300mL of 3M H₂SO₄ (93.0 g of 96.6 % sulphuric acid) before filtering by suction through # 42 ashless filter paper again.

The filtered product was dried for 2h at 110 °C and calcined at 500°C for 3 hours.

### ZrO₂/SO₄²⁻ Catalyst

Sulphated zirconium hydroxide was calcined at 550°C for 6 hours to obtain ZrO₂/SO₄²⁻ super acid catalyst

### EXAMPLE 1

Methanol was mixed with glycerol at a ratio of 4 to 1 by weight at ambient temperature. The mixture was pumped to a reactor packed with ZrO₂/SO₄²⁻ super acid catalyst synthesized as above. The reactor temperature was maintained at about 200°C and the reactor pressure was maintained at about 0.1 psia (5mm Hg). The reaction time (residence time based on the reactor volume/liquid feed rate) was 42.5 minutes. The glycerol in the feed was almost completely consumed (remaining in an amount in the order of 0.03 wt.% in the product), and the methanol consumed was 34.9 wt.% of the input. The products and other details are shown in Table 1 below:

**TABLE 1**

| Input | | Output (normalized to input) | | Compounds | Atmospheric boiling point (°C) |
|---|---|---|---|---|---|
| Unit | (g) | (g) | (%) | | |
| Methanol | 30.56 | 19.89 | 52.1 | Methyl alcohol | 66 |
| Glycerol | 7.64 | 0.01 | 0.03 | Glycerol | 290 |
| | | 0.70 | 1.8 | 1-Propene, 3-methoxy- | 35 |
| | | 0.68 | 1.8 | Propane, 1,1-dimethoxy | 83 |
| | | 0.93 | 2.4 | Acrolein dimethyl acetal | 90 |
| | | 2.76 | 7.2 | Water | 100 |
| | | 7.76 | 20.3 | 1,1,3-Trimethoxypropane | 145* |
| | | 1.60 | 4.2 | Dimethoxy-acetic acid methyl ester | 175* |
| | | 3.89 | 10.2 | Others (mostly esters) | |
| Total | 38.2 | 38.2 | 100.0 | | |

| | | | | | |
|---|---|---|---|---|---|
| * These boiling points were estimated using Riedel's Model. | | | | | |

### EXAMPLE 2

Methanol was mixed with glycerol at a ratio of 4 to 1 by weight at ambient temperature. The mixture was pumped to a reactor packed with ZrO₂/SO₄²⁻ super acid catalyst synthesized as above. The reactor temperature was maintained at 200°C and the reactor pressure was maintained at 0.1 psia (5mm Hg). The reaction time (residence time based on the reactor volume/liquid feed rate) was 4.3 minutes. The glycerol in the feed was almost completely consumed, and the methanol consumed was 28.9 wt.% of the input. The products and other details are shown in Table 2 below:

**TABLE 2**

| Input | | Output (normalized to input) | | Compounds | Atmospheric boiling point (°C) |
|---|---|---|---|---|---|
| Unit | (g) | (g) | (%) | | |
| Methanol | 34.2 | 24.03 | 56.3 | Methyl alcohol | 66 |
| Glycerol | 8.5 | 0.00 | 0.00 | Glycerol | 290 |
| | | 0.83 | 1.94 | 1-Propene, 3-methoxy- | 35 |
| | | 0.38 | 0.90 | Propane, 1,1-dimethoxy- | 83 |
| | | 1.14 | 2.66 | Acrolein dimethyl acetal | 90 |
| | | 3.27 | 7.67 | Water | 100 |
| | | 9.32 | 21.8 | 1,1,3-Trimcthoxypropane | 145* |
| | | 0.00 | 0.00 | Dimethoxy-acetic acid methyl ester | 175* |
| | | 3.73 | 8.73 | Others (mostly esters) | |
| Total | 42.7 | 42.70 | 100.0 | | |

| | | | | | |
|---|---|---|---|---|---|
| * These boiling points were estimated using Riedel's Model. | | | | | |

## Claims

1. A method of converting glycerol to one or more oxygenates of lower boiling point than glycerol, which comprises reacting glycerol with a lower alcohol in the vapor phase under reduced pressure at a reaction temperature in a range of 150 to 300°C in the presence of a solid acidic catalyst, wherein the lower alcohol is selected from the group consisting of methanol, ethanol, straight chained or branched propanol, straight chained or branched butanol and mixtures of any two or more thereof.

2. The method of claim 1, wherein said glycerol is a purified by-product from trans-esterification of triglycerides with alcohol.

3. The method of claim 2, wherein said by-product is purified by vacuum distillation.

4. The method of any one of claims 1 to 3, wherein said reduced pressure is 2.2 psia (115mm Hg) or less.

5. The method of any one of claims 1 to 4, wherein said reaction temperature is within a range of 200 to 300°C.

6. The method of any one of claims 1 to 5, wherein said catalyst is a superacid catalyst.

7. The method of claim 6, wherein said superacid catalyst is selected from the group consisting of zirconium sulphate on titanium oxide (TiO₂/Zr(SO₄)₂), sulphated tin oxide (SnO₂/SO₄²⁻) and sulphated zirconium hydroxide (ZrO₂/ SO₄²⁻).

8. The method of claim 6, wherein said solid acid catalyst is produced by calcining sulphated zirconium hydroxide to form a ZrO₂/SO₄²⁻ superacid catalyst.

9. The method of any one of claims 1 to 8, wherein said lower alcohol is used in a weight excess of 4:1 relative to said glycerol.

10. The method of any one of claims 1 to 9, wherein said reaction is carried out for a reaction time of 5 to 50 minutes.

11. The method of any one of claims 1 to 10, carried out in a manner to produce an oxygenated hydrocarbon product having an initial boiling point at atmospheric pressure of 250°C or less.

12. The method of any one of claims 1 to 11, carried out in a manner to produce an oxygenated hydrocarbon product containing at least one compound selected from the group consisting of 3-methoxy 1-propene, acrolein dimethyl acetal, 1,3-trimethoxypropane and 1,3-dimethoxy 2-propanol.

## Patentansprüche

1. Ein Verfahren zur Umwandlung von Glycerol in ein oder mehr Oxygenat(e) von niedrigerem Siedepunkt als Glycerol, welches das Reagieren von Glycerol mit einem niederem Alkohol in der Dampfphase unter reduziertem Druck bei einer Reaktionstemperatur im Bereich von 150 bis 300 °C in der Gegenwart eines azidischen Festkörperkatalysators umfasst, wobei der niedere Alkohol ausgewählt ist aus der Gruppe, bestehend aus Methanol, Ethanol, geradkettigem oder verzweigtem Propanol, geradkettigem oder verzweigtem Butanol und Mischungen von zwei oder mehr der Vorgenannten.

2. Verfahren nach Anspruch 1, wobei das Glycerol ein gereinigtes Nebenprodukt aus der Umesterung von Triglyceriden mit Alkohol ist.

3. Verfahren nach Anspruch 2, wobei das Nebenprodukt durch Vakuumdestillation aufgereinigt wird.

4. Verfahren nach einem der Ansprüche 1 bis 3, wobei der reduzierte Druck 2,2 psia (115mm Hg) oder weniger beträgt.

5. Verfahren nach einem der Ansprüche 1 bis 4, wobei die Reaktionstemperatur in einem Bereich von 200 bis 300 °C liegt.

6. Verfahren nach einem der Ansprüche 1 bis 5, wobei der Katalysator ein Supersäure-Katalysator ist.

7. Verfahren nach Anspruch 6, wobei der Supersäure-Katalysator ausgewählt ist aus der Gruppe, bestehend aus Zirkoniumsulfat auf Titanoxid (TiO₂/Zr(SO₄)₂), sulfatiertem Zinnoxid (SnO₂/SO₄²⁻) und sulfatiertem Zirkonium-Hydroxid (ZrO₂/SO₄²⁻).

8. Verfahren nach Anspruch 6, wobei der Säure-Festkörperkatalysator erzeugt wird durch Kalzinierung von sulfatiertem Zirkoniumhydroxid zur Bildung eines ZrO₂/SO₄²⁻ Supersäure-Katalysators.

9. Verfahren nach einem der Ansprüche 1 bis 8, wobei der niedere Alkohol in einem Gewichtsüberschuss von 4:1 bezogen auf das Glycerol verwendet wird.

10. Verfahren nach einem der Ansprüche 1 bis 9, wobei die Reaktion für eine Reaktionsdauer von 5 bis 50 Minuten ausgeführt wird.

11. Verfahren nach einem der Ansprüche 1 bis 10, wobei das Verfahren in einer Weise ausgeführt wird, um ein oxigeniertes Kohlenwasserstoffprodukt mit einem initialen Siedepunkt bei Atmosphärendruck von 250 °C oder weniger herzustellen.

12. Verfahren nach einem der Ansprüche 1 bis 11, wobei das Verfahren in einer Weise ausgeführt wird, um ein oxigeniertes Kohlenwasserstoffprodukt, enthaltend mindestens eine Verbindung ausgewählt aus der Gruppe, bestehend aus 3-Methoxy-1-Propen, Acrolein-dimethylacetal, 1,3-Trimethoxypropan und 1,3-Dimethoxy-2-Propanol, herzustellen.

## Revendications

1. Procédé de conversion du glycérol en un ou plusieurs composés oxygénés ayant un point d'ébullition plus bas que le glycérol, qui comprend la réaction du glycérol avec un alcool inférieur en phase vapeur sous pression réduite à une température de réaction dans une plage de 150 à 300 °C en présence d'un catalyseur acide solide, dans lequel l'alcool inférieur est choisi dans le groupe constitué par le méthanol, l'éthanol, le propanol à chaîne linéaire ou ramifiée, le butanol à chaîne linéaire ou ramifiée et des mélanges de deux quelconques ou plus de ceux-ci.

2. Procédé selon la revendication 1, dans lequel ledit glycérol est un sous-produit purifié de la transestérification de triglycérides avec de l'alcool.

3. Procédé selon la revendication 2, dans lequel ledit sous-produit est purifié par distillation sous vide.

4. Procédé selon l'une quelconque des revendications 1 à 3, dans lequel ladite pression réduite est de 2,2 psia (115 mm Hg) ou moins.

5. Procédé selon l'une quelconque des revendications 1 à 4, dans lequel ladite température de réaction est dans une plage de 200 à 300 °C.

6. Procédé selon l'une quelconque des revendications 1 à 5, dans lequel ledit catalyseur est un catalyseur superacide.

7. Procédé selon la revendication 6, dans lequel ledit catalyseur superacide est choisi dans le groupe constitué par le sulfate de zirconium sur l'oxyde de titane (TiO₂/2r (SO₄)₂) l'oxyde d'étain sulfaté (SnO_{2/}SO₄²⁻) et l'hydroxyde de zirconium sulfaté (ZrO₂/SO₄^{2*-*}).

8. Procédé selon la revendication 6, dans lequel ledit catalyseur acide solide est produit en calcinant de l'hydroxyde de zirconium sulfaté afin de former un catalyseur superacide ZrO₂/SO₄²⁻.

9. Procédé selon l'une quelconque des revendications 1 à 8, dans lequel ledit alcool inférieur est utilisé dans un excès de poids de 4 : 1 par rapport audit glycérol.

10. Procédé selon l'une quelconque des revendications 1 à 9, dans lequel ladite réaction est réalisée dans un temps de réaction de 5 à 50 minutes.

11. Procédé selon l'une quelconque des revendications 1 à 10, réalisé de façon à produire un produit d'hydrocarbure oxygéné ayant un point d'ébullition initial à la pression atmosphérique de 250°C ou moins.

12. Procédé selon l'une quelconque des revendications 1 à 11, réalisé de façon à produire un produit d'hydrocarbure oxygéné contenant au moins un composé choisi dans le groupe constitué par le 3-méthoxy 1-propène, le diméthylacétal d'acroléine, le 1,3-triméthoxypropane et le 1,3-diméthoxy 2-propanol.
